# EUROPEAN PATENT APPLICATION

(11) **EP 3 318 300 A1**
(43) Date of publication of application: **09.05.2018**
(21) Application number: 16196797.1
(22) Date of filing: 02.11.2016
(51) Int. Cl.: A61M 37/00, A61B 10/02

(54) **PERFUSION DEVICE FOR BIOLOGICAL TISSUE**

(71) Applicant: Miltenyi Biotec GmbH, 51429 Bergisch Gladbach (DE)
(72) Inventor: Fritzsch, Frederik, 51429 Bergisch Gladbach (DE); Miltenyi, Stefan, 51429 Bergisch Gladbach (DE); Poggel, Carsten, 51429 Bergisch Gladbach (DE); Stöters, Wolfgang, 51429 Bergisch Gladbach (DE); Bosio, Andreas, 51429 Bergisch Gladbach (DE); Kemmer, David, 51429 Bergisch Gladbach (DE); Adams, Timo, 51429 Bergisch Gladbach (DE); Mozon, Juan Antonio, 51429 Bergisch Gladbach (DE)
(74) Representative: Kisters, Michael Marcus

(57) **Abstract**

The invention is directed to an perfusion device for a biological tissue comprising a holder (1) and a plurality of hollow penetration structures (2), wherein the hollow penetration structures (2) are provided with a tip (3) capable of penetrating into the biological tissue and at least one orifice (4) which has fluid communication through the hollow penetration structure (2) and the holder (1) characterized in that the hollow penetration structures (2) consist of at least one polymer.

## Description

### BACKGROUND

The present invention is directed to a device for disaggregation of a biological tissue wherein the device is provided with hollow penetration structures which penetrate into the tissue and are configured to administer at least one agent to disaggregate the biological tissue.

Cells which are strongly interconnected to form a biological tissue like epithelial cells are difficult to isolate into single, living cells from the tissue. While it is possible to mechanically destroy the infrastructure of the biological tissue and isolate single cells from the resulting debris, the thus obtained yield of living, unharmed cells is rather low.

It is known to isolate cells from organs in a more gentle perfusion process, but this requires cumbersome perfusion of the organ through an appropriate blood vessel with a sequence of buffer solutions. Such processes are known for the isolation of cardiomyocytes, or hepatocytes etc.

The procedure to isolate intact hepatocytes was first introduced by Howard and Pesch (1967) J Cell Biol 35, 675-684 and refined by Berry and Friend (1969) J Cell Biol 43, 506-520; Seglen (1976) Methods Cell Biol. 13, 29-34 and Klaunig (1981) In Vitro 17, 913-925.

A similar but ex-vivo process is used for hepatocyte isolation from livers of bigger vertebrates and/or for the isolation of adult cardiomyocytes. In these applications, the target organ is excised before cannulating a blood vessel.

For the generation of human hepatocytes see review of Gomez-Lechon and Castell, "Isolation and culture of human hepatocytes" in Berry and Edwards, The Hepatocyte Review, (2000), 11-15. The tissue is put on ice as fast as possible and inspected for large vessels. Cannulae are inserted into the largest vessels of the cut surface and fixed with tissue glue. In a first step, the liver tissue is perfused extensively with an calcium-depleting buffer containing EGTA (or EDTA) at about 10ml/catheter. In the second step, it is perfused at the same flow rate with an about 0,2 U/ml collagenase-containing enzyme mix until the tissue shows irreversible deformation. Mostly, the enzyme buffer is recirculated to reduce costs. After digestion, the liver tissue is gently dispersed with a spatula. Analogues to the rodent procedure, the cell suspension is filtered and hepatocytes are enriched by a (optionally repeated) low-spin centrifugation.

Cardiomyocytes are generated by the so-called Langendorff perfusion system. The heart is rapidly excised from the body and, under a microscope, a cannula is carefully inserted into the aorta and fixed with a ligature. Then, the heart is perfused with a calcium-free buffer to arrest contraction, followed by a collagenase-based enzyme solution to digest the extracellular matrix of the heart. After digestion, the heart is mechanically dissociated with forceps and cardiomyocytes are dispersed into a single-cell suspension.

Lung alveolar epithelial cells are isolated in a similar procedure by inserting and fixing a cannula in the trachea and applying appropriate buffer and enzymes.

US20110295149 discloses a device to obtain cells from tissue by an abrasive extraction of surface cells. The device is fixed on the tissue by vacuum and the cells are cut from the tissue with an abrasive component. The such obtained cells may be further liquefied by appropriate enzymes. With this device, cells grown inside a tissue can only be harvested by destruction of the tissue and further purification steps to remove the not desired cells material.

In summary, the known isolation processes for cells from biological tissue are elaborate, time-consuming and are always executed by skilled personal.

In another technical field, it is known to inject pharmacological active compounds into tissue like skin through a plurality of cannulas. The cannulas can be assembled in arrays having a common input lumen, as for example disclosed in US6689103, US8349554B2, US8366677B2, US8708965B2, US2011/0213335A1, WO2014/047287A1 or EP2749306. These publications are silent on the dissociation of the tissue to generate single-cells.

### SUMMARY

It was therefore an object of the invention to provide a device capable of penetrating into a biological tissue and administering agents for disaggregation of the biological tissue, at best into isolated, living cells without the tedious and inconvenient need of cannulating a distinct lumen or cavity like a blood vessel.

It was found that a device comprising a plurality of injection sites can be used for administering fluids into a biological tissue for disaggregation of the biological tissue. In addition, such device can be manufactured from polymers, especially with a 3D printing process.

Object of the invention is therefore a perfusion device for a biological tissue comprising a holder (1) and a plurality of hollow penetration structures (2), wherein the hollow penetration structures (2) are provided with a tip (3) capable of penetrating into the biological tissue and at least one orifice (4) which has fluid communication through the hollow penetration structure (2) and the holder (1) wherein the hollow penetration structures (2) consist of at least one polymer.

Another object is a process for manufacturing the perfusion device as disclosed herein by injection molding, extrusion deposition printing, fused deposition modeling printing, stereolithography or photopolymer digital light processing.

A further object of the invention is a process for disaggregation of a biological tissue with the device of the invention wherein the biological tissue is penetrated at least in part by at least one of the hollow penetration structures (2) and at least one agent to disaggregate the biological tissue into target cells is administered through the hollow penetration structures (2) into the biological tissue.

### BRIEF DESCRIPTION OF THE DRAWINGS

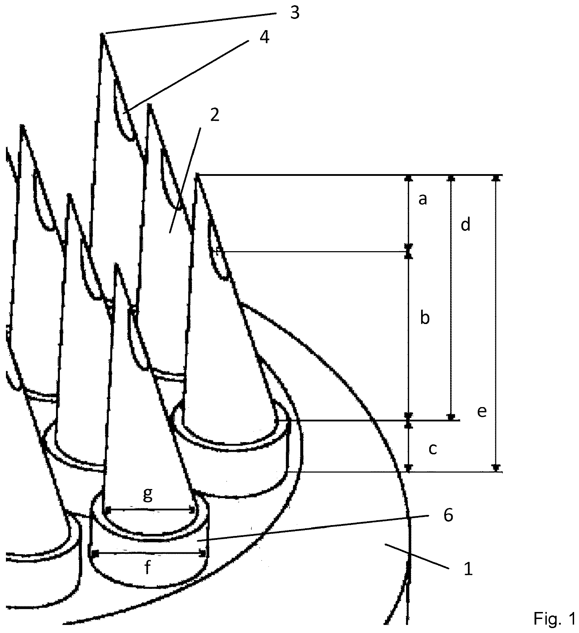

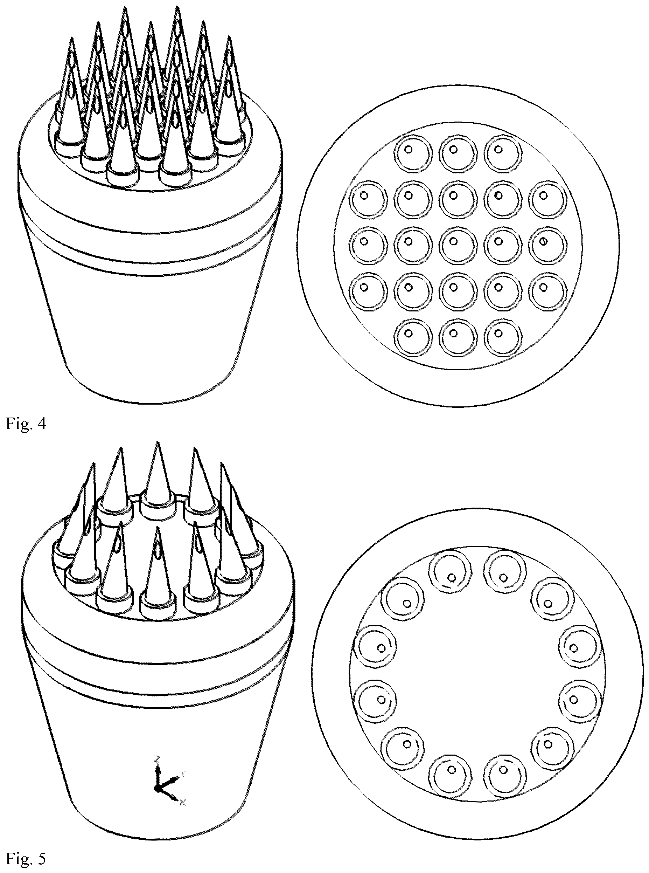

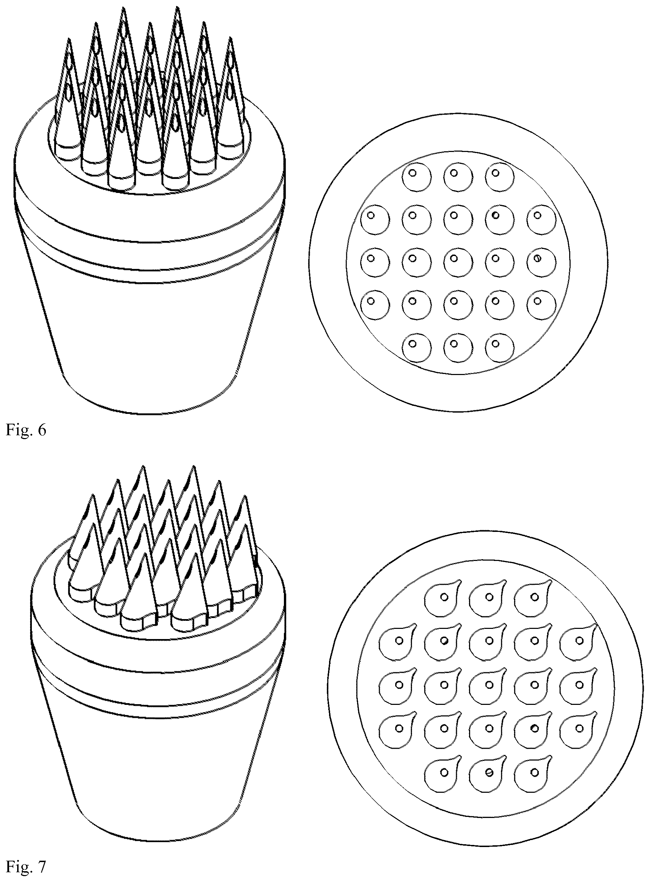

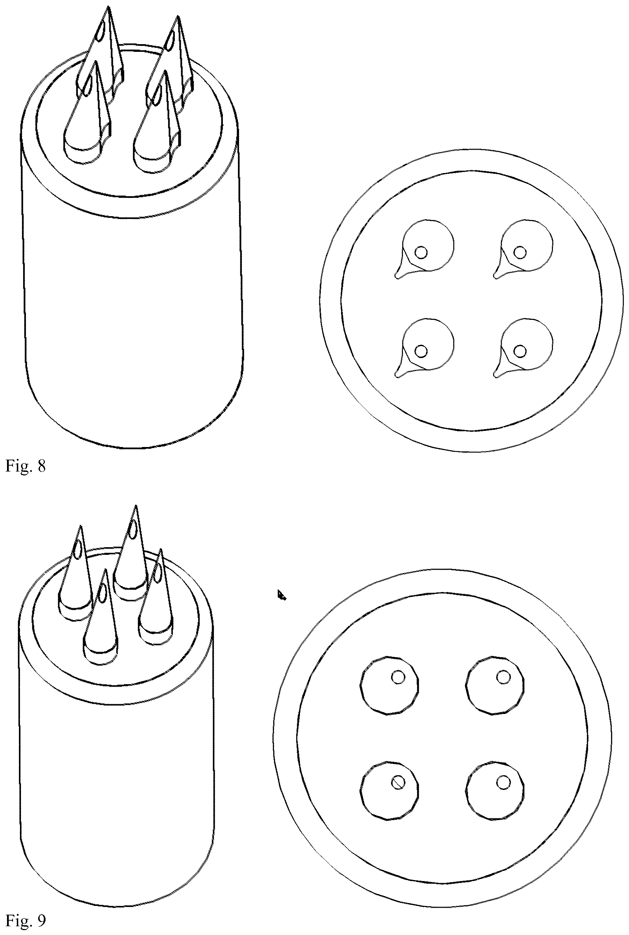

Fig. 1 to 11 show variants of a device of the invention, with (1) holder, (2) hollow penetration structure, (3) tip of hollow penetration structure, (4) center of orifice of hollow penetration structure, (5) fin of the hollow penetration structures, (6) stand of the hollow penetration structure, (7) orifice of the holder and (8) connector for tubing.

### DETAILED DESCRIPTION

In the following, the term "hollow penetration structures" and "needle" is used as synonym.

The term "hollow penetration structures" refers to any elongated object having for example, a needle-, cone- or pyramid-like shape, which is provided with a sharpened end and an orifice, wherein the orifice has a fluid communication through the structure.

The hollow penetration structures may be provided with a stand, i.e. a structure protruding from the body of the hollow penetration structure as marked with (6) in Fig.1. The optional stand is an integral part of the hollow penetration structure. If a stand is provided, the length of the hollow penetration structure includes the stand.

The device of the invention is shown in several embodiments in Fig. 1 to Fig. 11. In some figures, the marks are omitted but have the same meaning as in the figures with marks.

### Device of the invention

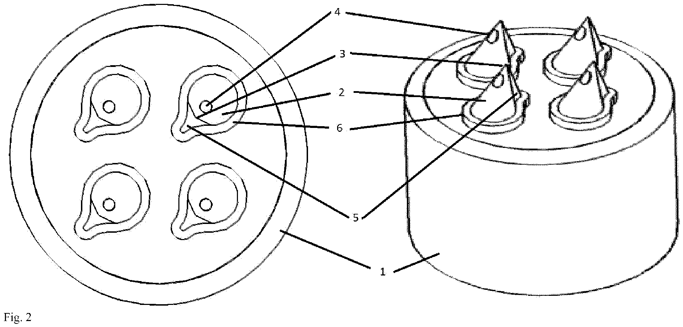

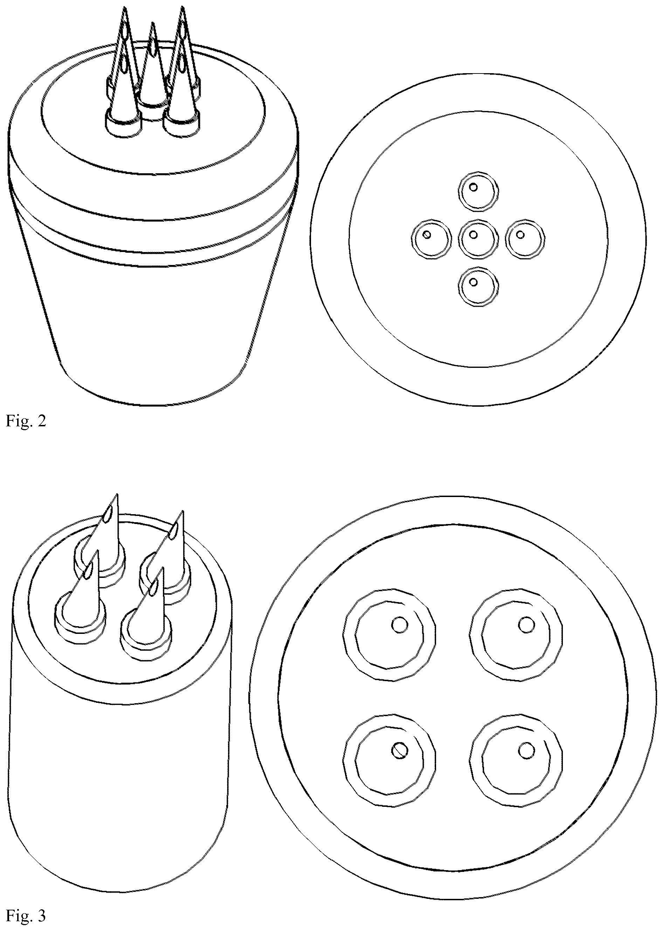

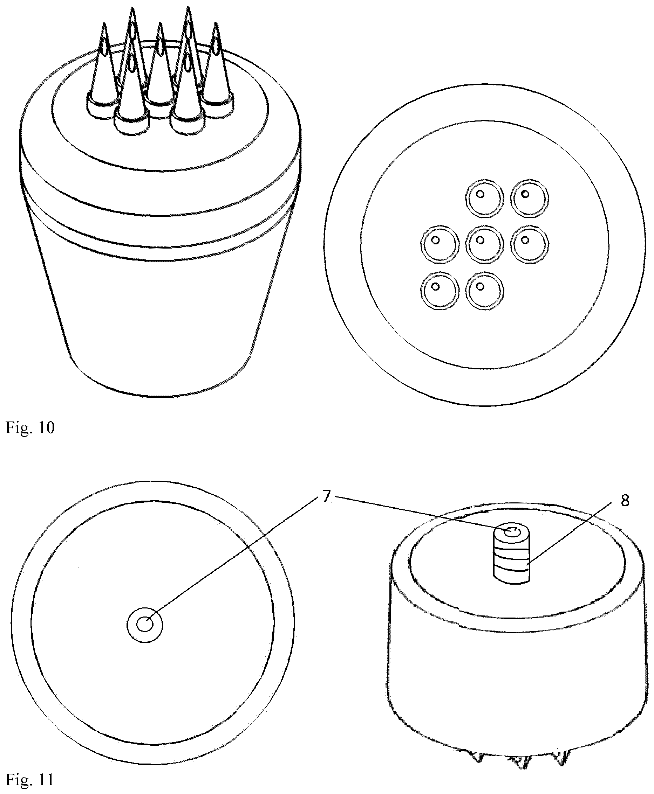

The perfusion device may by constructed having features of one or more of the following variants a) to i):
a) The center of the orifice of the hollow penetration structures (4) may be positioned at the tip (3) of the hollow penetration structure (2), similar to an injection needle.
b) The center of the orifice of the hollow penetration structures (4) may be positioned at a distance to the tip (3) of 5 - 50%, preferable 5 to 30% of the total length of the hollow penetration structure (2). As shown for example in Fig. 1, the center of the orifice of the hollow penetration structures (4) is positioned at a distance (a) to the tip (3) of 1,5 mm and at a distance (b+c) to the basis of the hollow penetration structure of 3,5 + 1,0 mm=4,5 mm. Given the total length of the hollow penetration structure (2) (e) with 1,5+ 3,5+1,0 =6 mm, (a) corresponds with 25% and (b+c) with 75%, respectively, of the total length of the hollow penetration structure.
c) The hollow penetration structures (2) may be provided with a fin (5) extending from the tip (3) to the base of the hollow penetration structure (2).
d) As shown for example in Fig. 2 (top view), the tip (3) can be located between the orifice (4) and the fin (5) of the hollow penetration structures (2). In this embodiment, the contour of the structures is not circular, but elongated in the direction of the fin.
e) As shown in Fig. 11, the holder (1) may be provided with at least one orifice (7, not shown in Figs 1- 10) having fluid communication to the hollow penetration structures (2). Fluid communication between the orifice of the holder (1) to the hollow penetration structures may be achieved for example by an appropriate number of channels connecting the orifice with each hollow penetration structure or by creating an volume within the holder with the orifice as input and the hollow penetration structures as output for fluids. As depicted in Fig. 11, the orifice of the holder (7) may be provided with a connector (8) for a tubing means, for example a Luer lock in order to attach standard tubing equipment to the device.
f) The hollow penetration structures (2) may be provided with a stand (6) at the basis of the hollow penetration structures (2). The stand is useful for sealing the tissue against the holder and to prevent leaking of fluids from the tissue. The stand may have different dimensions depending on the biological tissue, such as a height of 5 - 50%, preferable 10 - 25% of the total length of the hollow penetration structure (2), like 1 to 50 mm, preferable 1-5 mm. The diameter or cross-section of the stand (6) may be in its largest direction 105 to 200% of the diameter or cross-section of the hollow penetration structure (2) in its largest direction at its basis adjacent to the stand. For example as shown in Fig. 1, the stand may have a height (c) of 1,0 mm, corresponding to 16,6% of the total length of the hollow penetration structure (2) (e)= 1,5(a)+ 3,5(b)+1,0(c)=6,0 mm. Independently, the a diameter (f) of 2,5 mm as compared to the diameter at the basis of the hollow penetration structure (e) adjacent to the stand of 2,0 mm (=125% of e).
g) The hollow penetration structures of the present invention may be symmetrical or asymmetrical in view of its longitudinal axis. In the symmetric variant, the tip (3) is positioned in the middle of the structure as seen from above (in top view). In the asymmetric variants as shown in the drawings, the hollow penetration structures are asymmetrical shaped around an longitudinal axis, i.e. as seen from above (in top view), the tip (3) is positioned not in the middle of the structure. In a preferred variant, in top view, the tip (3) is located between the orifice (4) and the fin (5) of the hollow penetration structures (2).
h) The hollow penetration structures (2) may have a length (including the stand, where applicable) of 1 to 100 mm, preferably 2 to 20 mm, most preferably 4 to 5 mm and an outer diameter at its basis (directly adjacent to the holder, i.e. including the stand, where applicable) of 0,05 to 5 mm, preferably 0,2 to 1 mm, most preferably 0,3 to 0,7 mm and independently, an inner diameter of 0,02 to 4 mm, preferably 0,1 to 1 mm, most preferably 0,1 to 0,6 mm.
i) The number of hollow penetration structures (2) depends on the size of the biological tissue and may vary between 2 and 500, preferably between 5 and 100, and most preferably between 20 and 70. The hollow penetration structures (2) may be arranged in any geometry or array on the holder and may have the same or different length.

Depending on the size, thickness and the outer form of the biological tissue, the number, length, outer/inner diameter and area of hollow penetration structures (2) can be selected to achieve maximum effect of penetration.

Optionally, the hollow penetration structures (2) are provided with a means to stop the flow of reagents through a needle when the opening of a needle is placed not within the biological tissue, i.e. in case a needle did not penetrate into the tissue or penetrated through the tissue.

### Process of manufacture of the device

The device of the invention, i.e. the holder (1) and the hollow penetration structures (2) (including the optional stand) may be produced from the same or different material like polymers such as polyamide, polystyrene, polyolefins like polyethylene and polypropylene, polycarbonate, polyoxymethylene, polymethylmethacrylate, poly lactic acid, polyamides or steel.

The device of the invention may be manufactured by any method known to a person skilled in the art. For example, the perfusion device according to the invention may be produced by extrusion deposition printing of a support structure comprising water soluble polymers, then extrusion deposition printing of the surface structure comprising water in soluble polymers and removing the support structure comprising water soluble polymers by a solving in an aqueous medium.

Preferred methods are injection molding and 3D printing, for example by extrusion deposition, fused deposition modeling, stereolithography or photopolymer digital light processing.

A person skilled in the art is familiar with such 3D printing processes and the necessary equipment. A suitable 3D printer is for example M120 Scan-LED Printer from Innovation MediTech GmbH, with FotoMed® LED.A as printing photorestist polymer. Usually, layers of polymer having a thickness of 25 µm are cured by UV radiation with subsequent removal of the liquid uncured FotoMed® LED.A. by shaking. After the final layer is printed /cured, the uncured FotoMed® LED.A is removed by flushing the object in ultrasonic bath filled with isopropanol. Finally, post curing in N₂ atmosphere e.g. in the post curing unit PCU 90 (Innovation MediTech GmbH) may be performed to reach an appropriate shore hardness of the needles.

### Use of the device

In practical use, the biological tissue is pressed against the hollow penetration structures (or vice versa), resulting in penetration of the biological tissue at least in part by the hollow penetration structures (2).

The term "penetration" as used herein means that the needles are placed into the biological tissue in order to administer the release agent into the biological tissue. It is not desired to pierce or puncture the needles through the biological tissue since the disaggregation agent would then not or not sufficiently enter the biological tissue to disaggregate the biological tissue. It should be taken care in the process of the invention that the majority of the needles are placed inside the biological tissue and do not pierce or puncture through the biological tissue. At best, all needles are placed into the biological tissue at 30 - 70 %, preferable approximately 50% of its thickness.

In a variant, the biological tissue is either positioned on a support or the hollow penetration structures and the support is pressed against the hollow penetration structures (or vice versa), resulting in penetration of the biological tissue at least in part by the hollow penetration structures (2).

The support has the function of providing a mechanical counterpart to the hollow penetration structures when penetrating into the tissue. Insofar, the design of support is not essential and may be provided in form of a filter, mesh, rack, grid or even a plate with orifices (in order to prevent the tissue being soaked in fluids).

The support may be permeable for fluids, like the filter area of a filter cage, for example a "Smart Strainer" filter cage commercialized by Miltenyi Biotec GmbH

The perfusion device, according to the invention, may be used manually, i.e. with syringes and appropriate vessels, but can also be used in an automated environment comprising one or more pumps and tubing sets.

A further object of the invention is therefore a process for disaggregation of a biological tissue with a device as disclosed wherein the biological tissue is penetrated at least in part by at least one of the hollow penetration structures (2) and at least one agent to disaggregate the biological tissue into target cells is administered through the hollow penetration structures (2) into the biological tissue.

The agent to disaggregate the biological tissue is administered through the hollow penetration structures in form of a fluid. To this end, the holder (1) may be provided with at least one orifice ((7) in Fig. 11) having fluid communication to the hollow penetration structures (2). The orifice of the holder may be provided with a suitable connector ((8) in Fig. 11) like a Luer lock in order to attach standard tubing equipment to the device.

The process of the invention may be applied to a great variety of biological tissues. However, depending on the preparation and the macroscopic nature of the biological tissue, the target cells are disaggregated from the cellular infrastructure of the biological tissue by administering the agent, but cannot be released/extracted from the biological tissue and/or leave the biological tissue. This might occur if the outer structure of the biological tissue is still intact or if the biological tissue is provided with a capsule. e.g. an epithelial cell sheet. Extracting target cells from such biological tissue requires mechanically opening of the capsule.

In another embodiment of the process of the invention, after administering of at least one release agent into the biological tissue, the biological tissue is mechanically opened and the target cells are extracted from the biological tissue. In variants of this embodiment, the biological tissue is mechanically opened by cutting, piercing, or rupturing the biological tissue manually by appropriate tools. In another variant, the hollow penetration structures are used as cutting device, for example by rotating the holder against the tissue.

The process may comprise an additional step to remove unwanted "non-target cells", e.g. blood cells, before administering at least one agent to disaggregate the biological tissue. In this step non-target cells are extracted from the biological tissue by administering at least one washing fluid into the biological tissue. In this variant of the process according to the invention, the biological tissue is first washed with buffer to release non-target cells from the biological tissue.

In yet another variant, the agent to disaggregate the biological tissue and/or the buffer is repeatedly administered into the biological tissue, for example by a pump. In this variant, the fluids are recycled several times over/into the biological tissue, thereby improving yield.

The device of the invention can be used to isolate target cells from the various biological tissues. The thus isolated target cells may be used for research, diagnostics and cell therapy. For example, hepatocytes, cardiomyocytes or primary cells isolated with the device and the method of the invention can be used in assays to investigate either the cells themselves or their reactions with other cell types or analytes. Hepatocytes and cardiomyocytes are useful sources for toxicological screenings of chemicals, especially potential drug compounds. The device and method of the invention may further be used to isolate living cells for transplantation into injured organs or to create artificial organs or organoid-like structures.

### Target cells

The process of the invention can be applied to generate all type of target cells which are tissue-resident cells, especially cells from vertebrate or invertebrate tissue, preferably epithelial cells, endothelial cells, fibroblasts, myofibroblasts, hepatocytes, hepatic stellate cells, cardiomyocytes, podocytes, keratinocytes, melanocytes, neuronal cells including neurons, astrocytes, microglia and oligodendrocytes, leukocytes including dendritic cells, neutrophils, macrophages and lymphocytes, including T cells, B cells, NK cells, NKT cells and innate lymphoid type 1-3 cells, tissue stem cells including MSCs and progenitor cells of cells mentioned above.

### Biological tissue

The process of the invention can be applied to all types of biological tissue, like organs of vertebrates or invertebrates, preferably to spleen, heart, liver, brain and other neural tissues, kidney, lung, pancreas, breast, umbilical cord, skin, placenta, ovary, oviduct, uterus, prostate, tonsil, thymus, stomach, testis, trachea, cartilage, tendon, bone, skeletal muscle, smooth muscle, gut, colon, intestine, bladder, urethra, eye, gall bladder, organoids from cell cultures and tumors.

### Disaggregating agents

The term "disaggregating agent" as used herein means a fluid like a buffer comprising a substance used to destroy the anchorage of target cells within the tissue without influencing the target cells itself. This anchorage derives from interactions of the cells with the extracellular matrix or with adjacent cells. These interactions, e.g. tight junctions, gap junctions, desmosomes, and hemidesmosomes, are built mainly by proteins, e.g. cadherins, connexins, claudins and integrins, mostly in a calcium-dependent manner. Therefore, the release agent which destroys the tissue integrity may contain a calcium-free and/or a calcium-depleting agent and/or enzymes that degrade the extracellular matrix or extracellular protein-protein interactions. The administration of the components of the release agent may be sequentially or simultaneously.

For example, the agent to disaggregate the biological tissue (6) is selected from the group consisting of trypsin, chymotrypsin, papain, collagenase, elastase, dispase, thermolysin, hyaluronidase, clostripain and neutral protease from clostridium histolyticum, pronase, DNase I, pepsin, proteinase K, lysozyme, chelating agents for bivalent ions (like EDTA or citrate) and mixtures thereof.

Preferred is a sequestered application of a calcium-free or calcium-depleting buffer followed by an enzyme-containing buffer which degrades the extracellular matrix or extracellular protein-protein interactions. The calcium-depleting reagent may be a buffer containing EDTA, EGTA or citrate.

In another embodiments of the process according to the invention, a calcium-free buffer or a calcium-depleting buffer is used in a first step and a buffer containing calcium ions, preferably at least 50µM calcium ions, as well as a calcium-depending enzyme is used in a second step.

Most preferably, a calcium-free buffer or a calcium-depleting buffer is used in a first step and a buffer concentrate containing calcium ions as well as a calcium-depending enzyme is spiked into the first buffer as a concentrate in a second step.

As buffer, any aqueous fluid with pH values, osmolality and ion concentrations in the physiological range can be used. Preferred buffers are PBS, D-PBS, HBSS (Hanks' balanced salt solution), EBSS, DMEM (Dulbecco's Modified Eagle Medium), DMEM/F12, IMDM, RPMI, RPMI-1640, either in a complete form or in variants thereof, with or without phenol red, with or without HEPES, with or without glucose, optionally including a protein component like FCS (fetal calf serum), FBS (fetal bovine serum), HSA (human serum albumin), or BSA (bovine serum albumin). The pH of the buffer is between 6,0 and 8,0, preferably between 7,0 and 7,5.

The first buffer administered to the tissue might contain an anticoagulant like EDTA, EGTA, citrate or heparin to avoid blood coagulation within the tissue.

### Enzymes

In the process of the invention, a wide variety of enzymes, for example disclosed in Barry, Edwards and Barritt (1991) in Laboratory Techniques in Biochemistry and Molecular Biology Vol.21 can be used. The following enzymes may be used as component in the release agents:

Collagenase (EC 3.3.24.3) from Clostridium histolyticum, also called clostridiopeptidase A, is an enzyme mixture capable of causing hydrolytic cleavage of collagen molecules in their native conformation and at their helical region. These enzymes are very specific to collagen and have a specificity for the Pro-X- Gly-Pro (X = neutral amino acid) motive. They require Ca²⁺ for activity and usually contain Zn²⁺. Collagenase can also be isolated from many other bacterial sources (e.g. Achromobacter iophagus, Mycobacterium tuberculosis, Pseudomonas aeruginosa and other microorganisms). However, Clostridium histolyticum provides the main commercial source of collagenase. Crude preparations also contain contaminating protease activities like clostripain, neutral protease and further tryptic, caseinase and lipase activities. These can hydrolyse the portions of collagen which are not tightly wound in the helical form. Differences in the relative quantities of these contaminating activities are responsible for observed lot-to-lot variation.

Hyaluronidase (EC 3.2.1.35), an endoglycosidase with a specificity for endo-N-acetylhexosaminic bonds, usually derives from bovine and sheep testis. It hydrolyses 1,4 linkages between 2-acetacido-2-deoxy-ß-D-glucose and D-glucuronate residues in hyaluronic acid, a glycosaminoglycan found in the ground substance of virtually all connective tissue.

Trypsin (EC 3.4.21.4), a pancreatic serine protease, is capable of hydrolyzing peptides, preferentially at bonds involving the carboxyl group of the basic amino acids, L-arginine or L-lysine, and also shows some esterase and amidase activity. Purified trypsin is ineffective against native collagen and, therefore, not suitable for use by itself in the dissociation of adult liver tissue.

Clostripain (EC 3.4.22.8), like collagenase is derived from Clostridium histolyticum and highly specific for the carboxyl peptide bond of arginine. It is activated by calcium and found in commercial crude collagenase preparations.

Lysozyme (EC 3.2.1.17), an endoglycosidase, catalyses the hydrolysis of 1,4-ß-linkages between N-acetylmuramic acid and N-acetylglucosamine residues in glycosaminoglycans.

Pronase (EC 3.3.24.4), a non-specific protease isolated from Streptomyces griseus, hydrolyses nearly all naturally occurring peptide bonds.

Dispase (EC 3.4.24.28), a protease produced by Bacillus polymyxa, is capable of cleaving fibronectin, collagen IV, and to a lesser extent collagen I and hydrolyzing bonds involving leucine or phenylalanine.

Pancreatic Elastase (EC 3.4.21.36), or elastase 1, a serin protease, degrades elastin, an elastic fibre which, together with collagen, determines the mechanical properties of connective tissue.

Thermolysin (EC 3.4.24.27), a thermostable neutral metalloproteinase produced by Bacillus thermoproteolyticus, which requires Zn²⁺ for enzyme activity and four Ca²⁺ for structural stability, has a molecular weight of 34,6 kDa and specifically catalyzes the hydrolysis of peptide bonds containing hydrophobic amino acids.

Papain (EC 3.4.22.2), a cysteine protease, is an enzyme derived from papaya.

Chymotrypsin (EC 3.4.21.1), a digestive enzyme component of pancreatic juice, digests proteins and polypeptides in the duodenum and preferentially cleaves peptide amide bonds at aromatic amino acids (tyrosine, phenylalanine and tryptophan). After being synthesized as an inactive precursor (chymotrypsinogen), it is activated by trypsin cleavage.

Proteinase K (EC 3.4.21.64), a 28,9 kDa broad-spectrum serine protease produced by *Engyodontium album* (formerly *Tritirachium album*), is capable of digesting hair keratin. It is commonly used in molecular biology to digest proteins thus removing contaminations from nucleic acids preparations.

Pepsin (EC 3.4.23.1), whose zymogen (pepsinogen) is released by the chief cells in the stomach, degrades food proteins into peptides. It is most efficient in cleaving peptide bonds between hydrophobic and preferably aromatic amino acids such as phenylalanine, tryptophan, and tyrosine.

DNase I (EC 3.4.21.1) is a nuclease that cleaves DNA preferentially at phosphodiester linkages adjacent to a pyrimidine nucleotide, yielding 5'-phosphate-terminated polynucleotides with a free hydroxyl group on position 3', on average producing tetranucleotides. It acts on single-stranded DNA, double-stranded DNA, and chromatin. Regarding tissue dissociation, DNase I is useful for reducing cell aggregation caused by free DNA.

In one embodiment of the process according to the invention, a calcium-free buffer is used in a first perfusion cycle and a buffer containing significant amounts of calcium, preferably at least 50 µM, as well as a collagenase from Clostridium histolyticum, preferably between 0,05 and 1 Wünsch Units, is used in the second. In another embodiment of the process according to the invention, a calcium-depleting buffer is used in a first cycle and a buffer containing significant amounts of calcium, preferably at least 50µM, as well as a collagenase from Clostridium histolyticum, preferably between 0,05 and 1 Wünsch Units, is used in the second cycle.

### Washing fluid

The washing fluid used in the process of the invention may comprise an aqueous buffer with pH, osmolality and ion concentrations which are in the physiological range. Preferred buffers are PBS, D-PBS, HBSS (Hanks' balanced salt solution), EBSS, DMEM (Dulbecco's Modified Eagle Medium), DMEM/F12, IMDM, RPMI, RPMI-1640, either in a complete form or in variants thereof, with or without phenol red, with or without glucose, optionally including a protein component like FCS (fetal calf serum), FBS (fetal bovine serum), HSA (human serum albumin), or BSA (bovine serum albumin). The pH of the buffer is between 6,0 and 8,0, preferably between 7,2 and 7,4.

## Claims

1. Perfusion device for a biological tissue comprising a holder (1) and a plurality of hollow penetration structures (2), wherein the hollow penetration structures (2) are provided with a tip (3) capable of penetrating into the biological tissue and at least one orifice (4) which has fluid communication through the hollow penetration structure (2) and the holder (1) **characterized in that** the hollow penetration structures (2) consist of at least one polymer.

2. Perfusion device according to claim 1 **characterized in that** the hollow penetration structures (2) are elongated objects having a needle-, cone- or pyramid-like shape.

3. Perfusion device according to claim 1 or 2 **characterized in that** the orifice of the hollow penetration structures (2) is positioned at the tip (3) of the hollow penetration structure (2).

4. Perfusion device according to any of the claims 1 to 3 **characterized in that** the orifice of the hollow penetration structures (2) is positioned at a distance to the tip (3) of 5 - 50% of the total length of the hollow penetration structure (2).

5. Perfusion device according to any of the claims 1 to 4 **characterized in that** the hollow penetration structures (2) are provided with a fin (5) extending from the tip (3) to the base of the hollow penetration structure (2).

6. Perfusion device according to claim 5 **characterized in that** in top view, the tip (3) is located between the orifice (4) and the fin (5) of the hollow penetration structures (2).

7. Perfusion device according to any of the claims 1 to 6 **characterized in that** the hollow penetration structures (2) are provided with a stand having a height of 5 - 25% of the total length of the hollow penetration structure (2) and a diameter or cross-section in the largest direction of the hollow penetration structure (2) of 105 to 200% of the diameter or cross-section in its largest direction of the hollow penetration structure (2) at its basis adjacent to the stand.

8. Perfusion device according to any of the claims 1 to 7 **characterized in that** the holder (1) and the hollow penetration structures (2) consist of the same polymer.

9. Perfusion device according to any of the claims 1 to 8, **characterized in that** the hollow penetration structures (2) have a length of 1 to 100 mm.

10. Perfusion device according to any of the claims 1 to 9, **characterized in that** the holder (1) is provided with at least one orifice having fluid communication to the hollow penetration structures (2).

11. Process for manufacturing the perfusion device according to any of the claims 1 to 10 by injection molding, extrusion deposition printing, fused deposition modeling printing, stereolithography or photopolymer digital light processing.

12. Process for disaggregation of a biological tissue with a device according to any of the claims 1 to 10, **characterized in that** the biological tissue is penetrated at least in part by at least one of the hollow penetration structures (2) and at least one agent to disaggregate the biological tissue into target cells is administered through the hollow penetration structures (2) into the biological tissue.
